# EUROPEAN PATENT APPLICATION

(11) **EP 4 177 331 A1**
(43) Date of publication of application: **10.05.2023**
(21) Application number: 21832476.2
(22) Date of filing: 02.07.2021
(51) Int. Cl.: C12M 1/34, C12Q 1/6869

(54) **NUCLEIC ACID SEQUENCING METHOD AND APPARATUS**

(30) Priority: 02.07.2020 CN 202010632420
(71) Applicant: Geneus Technologies (Chengdu) Co., Ltd., Chengdu, Sichuan 610041 (CN)
(72) Inventor: ZOU, Yaozhong, Chengdu, Sichuan 610041 (CN); SU, Yunpeng, Chengdu, Sichuan 610041 (CN); QIN, Ronghuo, Chengdu, Sichuan 610041 (CN); JIANG, Peng, Chengdu, Sichuan 610041 (CN); LIU, Jiazhi, Chengdu, Sichuan 610041 (CN)
(74) Representative: Wenzel Nemetzade Warthmüller Patentanwälte Part mbB
(86) International application number: PCT/CN2021/104256
(87) International publication number: WO 2022/002251

(57) **Abstract**

A method for determining the type of a nucleotide on a nucleic acid sequence to be analyzed, and a nucleic acid sequencing method. In the method, at least one modified nucleotide for nucleic acid synthesis is separated from a nucleic acid sequence to be analyzed and other components on both sides of a membrane; when the modified nucleotide is transferred to the other side of the membrane under the action of an electric field by means of a nanopore embedded on the membrane, a synthesis reaction is conducted; moreover, the type of a base of the nucleotide is determined according to the change of the electrical properties of the nanopore in the process of the modified nucleotide is transferred by the nanopore, so as to implement sequencing.

## Description

### Field of the Invention

The present invention belongs to the field of nucleic acid sequencing, specifically, a technique for sequencing by synthesis of nucleic acids through a nanopore (Sequencing by Synthesis), and specifically relates to a nucleic acid sequencing method. In the method, at least one modified nucleotide for nucleic acid synthesis is separated from a nucleic acid sequence to be analyzed and other components on both sides of a bimolecular membrane, and the synthesis reaction is carried out when the modified nucleotide is transferred to the other side of the bimolecular membrane through a nanopore embedded in the bimolecular membrane under the action of voltage, and base type of the nucleotide is determined according to a change in electrical propertie of the nanopore caused by the transfer of the modified nucleotide by the nanopore, thus enabling sequencing.

### Background of the Invention

The concept of nanopore-based nucleic acid sequencing was introduced in 1995. Researchers have found that certain transmembrane proteins, such as the bacterial toxin α-hemolysin (α-hemolysin), can form stable channels of about 1 to 2 nm in diameter, called nanopores, in the phospholipid membrane. Single-stranded DNA (or RNA) molecules, due to their own charged nature, spontaneously cross the nanopore in an electric field and cause a change in the resistance of the nanopore during the crossing, generating the so-called blocking current. The four different bases of DNA (RNA), A, T(U), C and G, have recognizable differences in their blocking effects on the current generated when crossing the nanopore due to their own chemical structures, generating their corresponding characteristic blocking currents. Accurate detection of the characteristic blocking currents allows the determination of the corresponding base type and thus the nucleic acid sequence.

There are two main approaches to sequencing through nanopores that are commercially available. One approach, represented by Oxford Nanopore Technologies' system, allows single-stranded DNA molecules to pass directly through the nanopore and sequentially read the characteristic blocking currents corresponding to their bases. However, due to the small differences in the characteristic currents caused by different bases, multiple bases can stay in the nanopore at the same time making the blocking current characterization very complicated, which puts extremely high demands on the current data analysis at the later stage of sequencing. More importantly, this system has insurmountable difficulties in the determination of DNA sequences (homopolymer) of consecutive identical bases. Another approach is represented by the system used by Genia Technologies (currently part of Roche Sequencing Solutions), which uses modified nucleotide analogs to perform sequencing in parallel with nucleic acid synthesis. Although the labeling of nucleotides for replication can improve the recognition of the characteristic blocking currents corresponding to different bases, and the time interval between the entry of individual nucleotide labels into the nanopore can help to determine the sequences of consecutive homopolymers of the same bases, it is difficult to ensure that the label of each nucleotide used for synthesis enters the nanopore to give blocking current, which causes deletion error in the sequencing process; it is also difficult to avoid various background noises caused by the nucleotide label not participating in the synthesis reaction entering the nanopore to interfere with the reading of the valid signal, or even to generate the error of redundant blocking current signals being read by the system as valid sequence signals (insertion error). Compared to these two existing approaches, the present invention proposes a new sequencing improvement by using a nanopore to transport the nucleotides used for synthesis, thus ensuring that each nucleotide added to the nascent nucleic acid chain necessarily records its characteristic blocking current through the nanopore, thus avoiding deletion errors. Also, after a possible contact with the polymerase, the unreacted intact phosphate deoxynucleotide and the polyphosphate group produced by the reaction will pass through the nanopore again, and the unreacted intact phosphate deoxynucleotide and the polyphosphate group will give different blocking currents, thus helping to determine whether a particular deoxynucleotide has been incorporated into the nucleic acid product. This information will effectively avoid insertion errors caused by the system incorrectly recording signals from nucleotides not involved in the synthesis reaction.

### Summary of the Invention

One aspect of the present invention provides a method for determining type of a nucleotide on a nucleic acid sequence to be analyzed, comprising the steps of:
S01 providing at least one nucleotide molecule in a first compartment and a nucleic acid sequence to be analyzed in a second compartment, with the first compartment and the second compartment separated by a membrane having at least one nanopore;
S02 applying an electric field to drive the nucleotide molecule and/or a part thereof to pass through the nanopore in a first direction or to be inserted in the nanopore;
S03 measuring a first current characteristic value passing through the membrane in the state of S02 to identify the nucleotide molecule and/or a part thereof;
S04 applying an electric field in a direction opposite to that of the electric field applied in step S02 or in the same direction but with a lower driving voltage, causing the nucleotide molecule and/or a part thereof to pass through the nanopore and/or exit the nanopore in a second direction, with the second direction being opposite to the first direction;
S05 measuring a second current characteristic value passing through the membrane to identify the nucleotide molecule and/or a part thereof, comparing the second current characteristic value with a pre-determined standard current characteristic value of the nucleotide molecule or polyphosphate molecule in the state of S04 to determine whether the nucleotide molecule and/or a part thereof is attached to the nucleic acid sequence to be analyzed, and thus determining the type of nucleotide on the nucleic acid sequence to be analyzed in the second compartment.

In some embodiments, the term "lower driving voltage" in step S04 refers to that the electric field with a lower voltage has a voltage difference of 0 mV or more compared to the electric field with a higher voltage, for example, a voltage difference of 0 mV or more, 5 mV or more, 10 mV or more, 20 mV or more, 30 mV or more, 40 mV or more, 50 mV or more, 60 mV or more, 70 mV or more, 80 mV or more, 90 mV or more, 100 mV or more, 110 mV or more, 120 mV or more, 130 mV or more, 140 mV or more, 150 mV or more, 160 mV or more, 170 mV or more, 180 mV or more, 190 mV, 200 mV or more, or any value in any of the above voltage difference ranges.

In some embodiments, there is also provided in the second compartment including, but not limited to, a nucleic acid polymerase and a template primer, etc. In some preferred embodiments, the nucleic acid polymeras is bound to the nanopore.

In some embodiments, the nucleotide molecule is a modified nucleotide molecule as shown in Formula I:

NXP-L-B Formula I

wherein NXP (Nucleoside phosphate) represents a phosphate deoxyribonucleotide and/or a phosphate nucleotide, the phosphate deoxyribonucleotides comprising dAXP (deoxyadenine phosphate), dTXP (deoxythymidine phosphate), dCXP (deoxycytidine phosphate) and dGXP (deoxyguanine phosphate); the phosphate nucleotides comprising AXP (guanine phosphate), TXP/LTXP (thymine/uracil phosphate), CXP (cytosine phosphate) and GXP (guanine phosphate).

L represents a linker part having a long chain segment for spacing the NXP part and the B part of Formula I. In some preferred embodiments, the long chain segment is a biocompatible straight chain polymer, and such biocompatible straight chain polymer is for example, but not limited to, a PEG chain, a polymer formed by phosphodiester bonds (similar to nucleic acid backbone structure), a polypeptide, and suitable nanowires. The linker part also has a linking segment such as, but not limited to, biotin, maleimide, etc. for specific attachment of a bulky steric hindrance part.

B represents a bulky steric hindrance part, which is selected from a protein molecule, preferably a globular protein molecule. The bulky steric hindrance part is characterized by a structurally stable nature and is linked to the linker part by the above-mentioned linking segment, such as, but not limited to, a biotin-binding protein (avidin), which is linked to the linker by biotin; or xylanase with a cysteine residue introduced at the C-terminus, which is linked to the linker via maleimide.

In some preferred embodiments, the biocompatible straight chain polymer segment is preferably a polyethylene glycol PEG or a polypeptide comprising glycine and serine (e.g. GSGSGGGSSSSSSSGSSSSSSGSSS...). In some preferred embodiments, the linking segment is biotin or maleimide. In some preferred embodiments, the bulky steric hindrance part is preferably a biotin-binding protein (avidin), which is linked to the linker by biotin; or a xylanase with a cysteine residue introduced at the C-terminus, which is linked to the linker via maleimide.

In some embodiments, the membrane is selected from a natural lipid bilayer membrane and a bilayer membrane formed by an artificial amphiphilic molecule. In some embodiments, the membrane has at least one nanopore. In some preferred embodiments, the membrane has one nanopore.

In some embodiments, the nanopore is selected from natural protein nanopores and nanopores prepared from artificial materials. In some embodiments, the nanopore is bacterial toxin α-hemolysin. In some embodiments, the nanopore is other type of protein pores, such as but not limited to MspA, CsgG, OmpF, etc. In some embodiments, the nanopores is a variant of wild-type protein nanopore with modifications. In some embodiments, the nanopore may also be an artificial nanopore prepared from an artificial material such as, but not limited to, silicon or graphene. The artificial material such as, but not limited to, silicon or graphene is made into a membrane, and then a nanopore is formed on the membrane.

Another aspect of the present invention provides a sequencing method for a nucleic acid sequence to be analyzed, comprising repeating steps S01 to S05 of the method according to the above aspect, sequentially determining type of each nucleotide of the nucleic acid sequence to be analyzed until the sequencing of the nucleic acid sequence to be analyzed is completed.

Another aspect of the present invention provides a nucleic acid sequencing device for the above described method for determining type of a nucleotide on a nucleic acid sequence to be analyzed and/or a nucleic acid sequencing method, comprising:
(a) a first compartment and a second compartment separated by a membrane having at least one nanopore;
(b) means for applying an electric field across the membrane; and
(c) components used to measure a current flowing through the membrane.

In some embodiments, the device of the present invention is a single-channel device based on the sequencing method of the present invention, intended to further illustrate the principles and implementation of the present invention. In practical applications, the present invention can also be extended to devices with multi-channel arrays for simultaneous sequencing of several DNA molecules.

### Brief Description of the Drawings

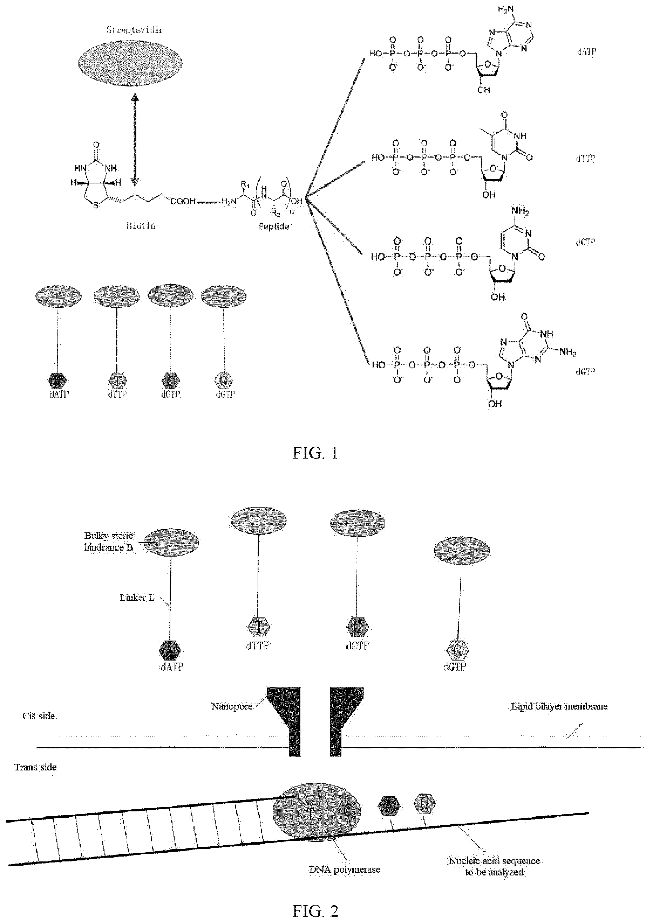

FIG. 1 illustrates a specific example of a modified nucleotide molecule according to the present invention, specifically deoxyribonucleotides triphosphate (dATP, dTTP, dCTP and dGTP) are linked to the polypeptide sequence and form a tight triphosphate nucleotide complex through the biotin moiety and the biotin-binding protein, respectively. A sketch of the four nucleotide molecular complexes is shown in the lower left corner of FIG. 1.
FIG. 2 illustrates a first compartment and a second compartment separated by a membrane having at least one nanopore according to the present invention, a modified nucleotide molecule is provided in the first compartment, and a nucleic acid sequence to be analyzed, a nucleic acid polymerase, and a template primer are provided in the second compartment, wherein the nucleic acid polymerase is bound to the nanopore.

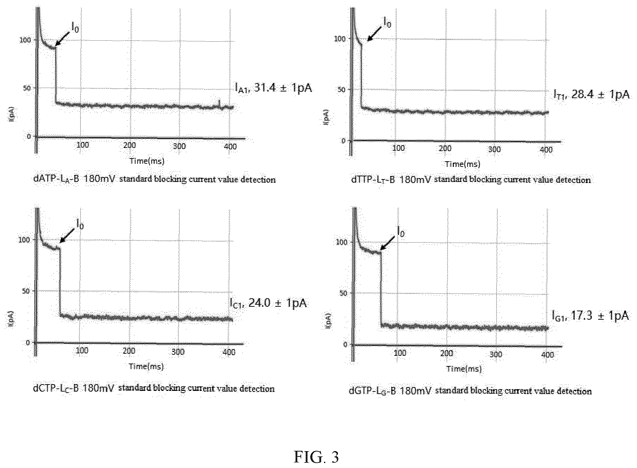

FIG. 3 illustrates representative current profiles and standard blocking current characteristic values I_{N1} for different complex molecules dATP-L_{A}-B, dTTP-L_{T}-B, dCTP-L_{C}-B and dGTP-L_{G}-B, upon interaction with nanopore after the nucleotides of the dNTP-L_{N}-B complexes are driven from the first compartment into the second compartment at a high voltage (180 mV).

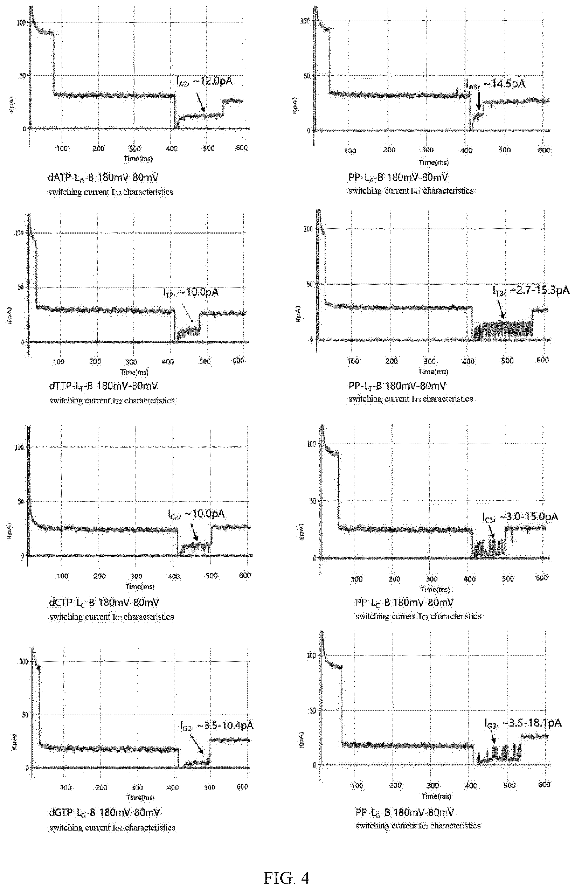

FIG. 4 illustrates the characteristic current profiles I_{N2} and I_{N3} corresponding to different molecules during the return of dNTP-L_{N}-B complex and PP-L_{N}-B from the second compartment to the first compartment after switching to a low voltage (80 mV) after the nucleotide enters the second compartment from the first compartment at 180 mV. For complexes with the same linker, I_{N2} and I_{N3} can be clearly distinguished.

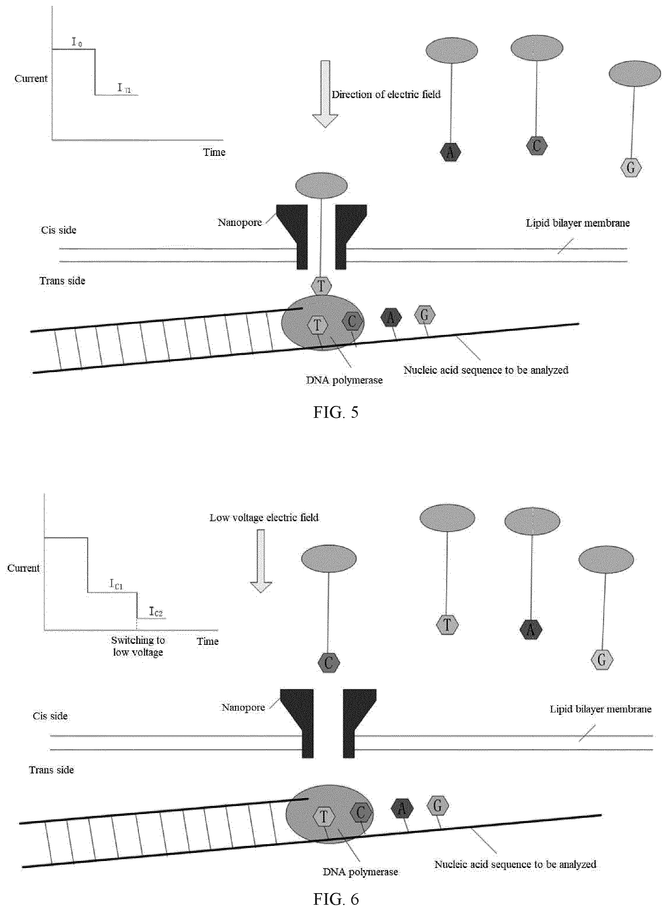

FIG. 5 illustrates that according to the present invention an electric field is applied so that the modified nucleotide molecule is inserted in the nanopore in a first direction and its nucleotide part passes through the nanopore into the second compartment, the nucleotide molecule and its corresponding linker part interacts with the nanopore and the measurement gives the corresponding current characteristic value. The type of nucleotide entering from the first compartment to the second compartment can be determined by comparing the current characteristic value obtained from the measurement with the standard blocking current characteristic value I_{N1} for the different nucleotides. FIG. 5 illustrates that the measured current characteristic value is consistent with I_{T1}, thus it can be determined that the nucleotide type is T.

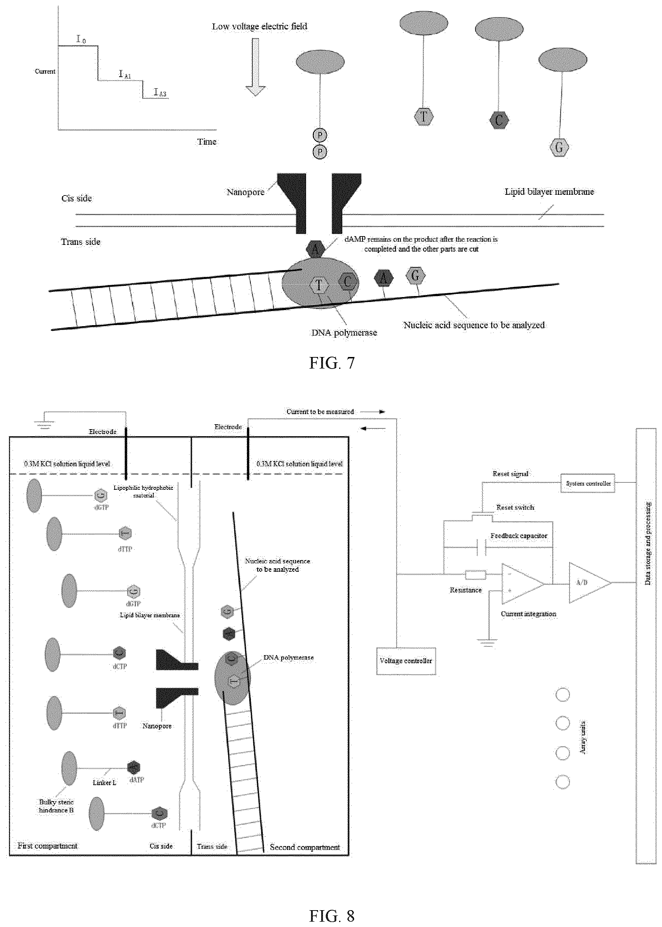

FIGs. 6-7 illustrate that according to the present invention, the system switches to a low-voltage electric field (such as 80 mV) after a certain period of time, and the driving force of the electric field is difficult to overcome the diffusion tendency of nucleotide molecules, the linker carrying intact nucleotide or pyrophosphate group after the enzymatic reaction passes through or exits the nanopore back to the first compartment in the second direction, respectively, during which the corresponding characteristic value of the blocking current is recorded. When the current characteristics match the I_{N2} characteristics, it can be determined that the linker carries a complete nucleotide and no synthesis reaction is carried out in the second compartment, such as the case shown in FIG. 6. When the current characteristics match with the I_{N3} characteristics, it can be determined that the linker carries only a pyrophosphate group (PP) on it, and the synthesis reaction catalyzed by polymerase has occurred in the second compartment, and the base previously determined by I_{N1} is the complementary base on the nucleic acid template in the second compartment, such as the case shown in FIG. 7, where it can be determined that the nucleotide entering the second compartment from the first compartment is A and that the DNA synthesis reaction is complete and the corresponding position on the template is T.
FIG. 8 is a schematic diagram of structure of the nucleic acid sequencing device of the present invention.

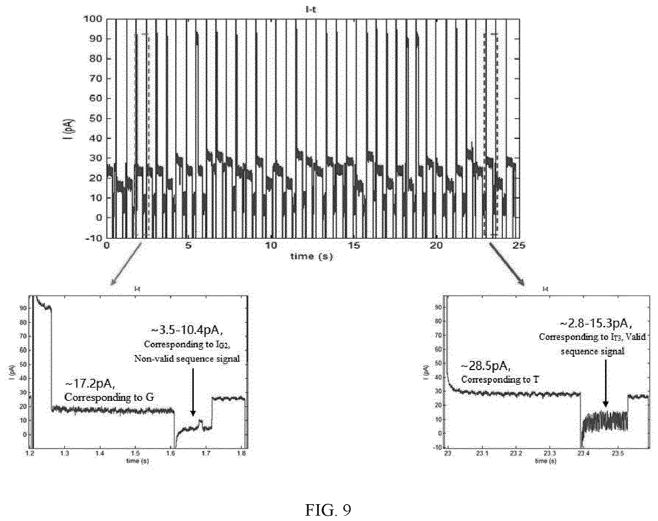

FIG. 9 shows a graph of the current signal obtained when sequencing was performed. In the lower left local amplification region 1, the nucleotide entering the second compartment was determined to be G based on the current characteristic value at 180 mV, but after switching to a low voltage of 80 mV, its current characteristic corresponded to I_{G2}, indicating that the intact nucleotide was on the linker and no synthesis reaction occurred in the second compartment, so it was judged that G was not a valid sequence signal here. In the lower right local amplification region 2, the nucleotide entering the second compartment was determined to be T based on the current characteristic value at 180 mV, and after switching to a low voltage of 80 mV, its current characteristic corresponded to I_{T3}, thus the synthesis reaction was completed, and C was judged to be a valid sequence signal here.

### Detailed Description of the Invention

It should be understood that the different applications of the methods disclosed herein may vary according to the specific needs of the art. It should also be understood that the terms used herein are intended to describe specific embodiments of the invention only and are not intended to be limiting.

The present invention provides a method for determining the type of nucleotide on a nucleic acid sequence using a nanopore on a bilayer membrane and modified nucleotides. The present invention also provides a sequencing method for a nucleic acid sequence using a nanopore on a bilayer membrane and modified nucleotides. The modified nucleotides are complexes as shown in Formula I:

NXP-L-B Formula I

wherein NXP (Nucleoside phosphate) represents a phosphate deoxyribonucleotide and/or a phosphate nucleotide, the phosphate deoxyribonucleotides comprising dAXP, dTXP, dCXP and dGXP; the phosphate nucleotides comprising AXP, TXP/UXP, CXP and GXP.

As used herein, the term "phosphate deoxynucleotide" includes, but is not limited to, monophosphate deoxynucleotides and polyphosphate deoxynucleotides, wherein polyphosphate deoxynucleotides include, but are not limited to, diphosphate deoxynucleotides, triphosphate deoxynucleotides, tetraphosphate deoxynucleotides, pentaphosphate deoxynucleotides and hexaphosphate deoxynucleotides, preferably triphosphate deoxynucleotides. For the purposes of the present invention, the term "phosphonucleotide" includes, but is not limited to, monophosphate nucleotides and polyphosphate nucleotides, wherein polyphosphate nucleotides include, but are not limited to, diphosphate nucleotides, triphosphate nucleotides, tetraphosphate nucleotides, penta-phosphate nucleotides and hexaphosphate nucleotides, preferably triphosphate nucleotides.

The phosphoribonucleotides and/or phosphodeoxynucleotides employed in the present invention can also be modified at multiple sites, such as, but not limited to, the second position on ribose, the fifth position on pyrimidines, and the seventh position on purines. Any manner of modification, as long as the modified phosphate nucleotide and/or phosphate deoxynucleotide can still support efficient nucleic acid synthesis reactions, can be a candidate modification option in the present invention to help obtain better sequencing results. According to their corresponding blocking currents, appropriate modifications can be screened to make the characteristic blocking currents of different nucleotides more distinguishable and the signals clearer and easier to be identified. In addition to this, modifications can also alter the size, or charge characteristics, of phosphonucleotide and/or phosphodeoxynucleotide molecules, thereby slowing their entry, transit and/or exit times into the nanopore, helping to obtain clearer and more accurate current measurements.

In the method of the present invention, the nucleic acid sequence to be analyzed may be a DNA nucleic acid sequence or an RNA nucleic acid sequence. When determining the type of nucleotide in a DNA nucleic acid sequence or sequencing a DNA nucleic acid sequence, either DNA polymerase can be used to employ modified deoxyribonucleotides (dATP, dTTP, dCTP, and dGTP) or RNA polymerase can be used to synthesize an RNA strand using a DNA template and modified nucleotides (ATP, TTP/LTTP, CTP, and GTP). When determining the nucleotide type in the RNA nucleic acid sequence or sequencing the RNA nucleic acid sequence, reverse transcriptase can be used to synthesize a cDNA strand complementary to it using the above-mentioned deoxyribonucleotides as a template.

L represents a linker part having a long chain segment and a linking segment.

The long chain segment is preferably a biocompatible straight chain polymer. Biocompatible straight chain polymers that can be used in the present invention include, but are not limited to, straight chain polypeptides, preferably straight chain polypeptides. As the main structure of the long chain segment itself, in addition to straight chain polypeptides, other chemical chain polymers such as polymers formed by phosphodiester bonds (similar to the main chain structure of nucleic acids), polyethylene glycol PEG, and suitable nanowires, etc., can also be used in the present invention. The length range of the long chain segment can be adjusted according to the scale of the nanopore protein chosen and the different ways of coupling the nanopore and the nucleic acid polymerase. In the present invention, the long chain segments may have a length range in the range of 5 to 25 nm, preferably in the range of 8 to 15 nm.

In the present invention, different linkers can be distinguished by subscript letters, such as L_{N}, L_{A}, L_{T}, L_{U}, L_{C}, L_{G}, etc., where the subscripts A, T, U, C and G represent the type of nucleotide linked to the linker part L, and N is a generic representation of the nucleotide, which can be any one of A, T, U, C and G.

The linking segment that can be used in the present invention is for example, but not limited to, biotin, maleimide, etc. The linking segment has the ability to bind specifically to a specific protein, for example biotin binds specifically to biotin-binding protein.

B represents a bulky steric hindrance part, and as used herein, the term "bulky steric hindrance part" refers to a biomolecular part having a diameter greater than 1 to 2 nm, preferably greater than 2 nm, and more preferably greater than 3 nm. The bulky steric hindrance part is selected from a protein molecule, preferably a globular protein molecule. The bulky steric hindrance part is characterized by a structurally stable nature and is linked to the linker part by the above-mentioned linking segment, such as, but not limited to, a biotin-binding protein, which is linked to the linker by biotin. In addition to the use of biotin/biotin-binding protein to couple proteins at the end of the linker chain, the use of other proteins or other blockers with diameters larger than the nanopore, and other coupling methods are also applicable to the present invention. For example, a maleimide active group is added to the end of the linker by chemical synthesis as a linking segment, and then any stable, spherical protein with a size larger than the nanopore pore size is selected. For example, in some preferred embodiments, xylanase is employed as the bulky steric hindrance part, and cysteine is introduced at the C-terminus of the recombinant protein, which is covalently coupled to the linking segment maleimide using its sulfhydryl group -SH.

After said modified nucleotide molecule passes through the nanopore or is inserted in the nanopore, said linker allows the phosphonucleotide part to be confined near the opening of the nanopore. In addition, the linker assists in controlling the reverse passage time of the phosphonucleotide or phosphate group through the nanopore. The linker can be chemically modified to introduce charged groups, such as -NH₂ group carrying a positive charge or -PO₄ group carrying a negative charge in the system's operating pH range (6-9). The variation of the charge density and charge sites introduced on the chain allows the entire modified nucleotide molecule, i.e., the phosphonucleotide/phosphate deoxynucleotide-linker-bulky steric hindrance molecule complex, to be subjected to different driving forces in the electric field, thus affecting the timing and manner of interaction of the nucleotide part with the nanopore, as well as the direction of motion at a given voltage. For example, introducing a positive charge near the site where the linker is attached to the phosphate nucleotide reduces the net negative charge density of the complex, allowing the nucleotide to be driven by a reduced electric field in the nanopore, thus allowing it to exit the nanopore in the second direction even at high voltages, facilitating the recording of the current characteristics at high voltages when exiting the nanopore and improving the signal-to-noise ratio.

As used herein, the term "lipid bilayer" refers to a membrane prepared based on the tendency of lipid molecules, such as but not limited to phospholipids, to form stable lipid bilayers in the aqueous phase. The lipid bilayer that may be used in the present invention includes, but is not limited to phospholipid bilayers.

As used herein, the term "nanopore" refers to a nano-sized pore which can be formed by pores in porous proteins or synthetic materials such as silicon or graphene. Protein pores in electrically insulating membranes or artificial solid pores machined from insulating materials can be used as single molecule detectors. It can be a bioprotein channel in a highly resistive lipid bilayer, a protein channel in a synthetic membrane or directly an artificial solid state pore. Two general approaches exist for the preparation of nanopores for nucleic acid analysis: (1) Organic nanopores prepared from naturally occurring molecules, such as α-hemolysin pores. (2) Synthetic solid-state nanopores generated by several conventional and unconventional fabrication techniques.

As used herein, the terms "nucleotide" and "NXP" cover both nucleotides and deoxyribonucleotides, unless the context specifically indicates otherwise.

In some embodiments, the sequencing system of the present invention involves the formation of a phospholipid bilayer for separating and insulating the cis and trans sides of the bilayer and providing a suitable chemical environment for the nanopore to embed, forming a small pore of 1.4 to 2 nm diameter to pass through the cis and trans sides, thereby allowing ions to pass through to form microcurrents under the action of an electric field. Using, for example, but not limited to, the phospholipid 1,2-diphytanoyl-sn-glycero-3-phosphocholine (DPhPC) or other amphiphilic compounds, there are currently a variety of available methods, such as coating, folding, microfluidic methods, etc., for forming phospholipid bilayers on scales of several microns to 200 microns across surface materials with hydrophobic properties, such as polyimide, polyethylene, etc. These methods have been reported in numerous literature sources describing the materials and steps for their specific implementation, and will not be repeated in the present invention.

The present invention is quite adaptable and can theoretically employ a variety of different transmembrane nanopores for the transport and recognition of phosphonucleotides, such as but not limited to α-hemolysin, γ-hemolysin, MspA, OmpF, etc. Nanopores can be used for sequencing in the manner described herein as long as they are structurally stable after being embedded in phospholipid membranes, the corresponding currents are relatively stable and measurable, and the pore size is appropriate to allow the passage of phosphonucleotides and linkers while blocking large-sized proteins. In addition to wild-type nanopore proteins, the present invention can also employ modified variants of nanopore proteins. Finally, in addition to protein nanopores, the present invention is equally applicable to artificial solid-state nanopores as long as they meet the prerequisites of current stability and appropriate pore size.

By applying a voltage of, for example, 50-300 mV, such as 75 mV-275 mV, 100 mV-250 mV, 125 mV-225 mV, 150 mV-200 mV, or 80 mV, 90 mV, 100 mV, 125 mV, 150 mV, 175 mV, 180 mV, 190 mV, 200 mV, or any value between any of the above voltage values, to both sides of the insulating lipid bilayer, the system will form a potential gradient across the membrane along the inner diameter of the nanopore. The electrolyte is driven by the potential gradient to form a current in the nanopore by directed motion within the nanopore, i.e., the open nanopore current. When the negatively charged nucleotide part approaches the nanopore opening under the dual action of free diffusion and electrophoretic motion, it will be captured by the potential gradient along the nanopore and thus driven by the electric field force through the narrow nanopore to achieve transmembrane transport. In this process, the nucleotides will interfere to some extent with the movement of the electrolyte in the nanopore, thus changing the current magnitude and creating a so-called blocking current. The four different bases A, T(U), C, and G of different nucleotides can be distinguished from each other by the blocking currents formed by their interaction with nanopores due to differences in structure and physicochemical properties, as well as in linker structure. Recording and identifying this characteristic blocking current can be used to determine the type of nucleotide bases passing through the nanopore.

In the method of the present invention, the modified nucleotide part connected to the linker passes through the nanopore under the action of a strong electric field, but the bulky steric hindrance part connected to the linker cannot pass through the nanopore due to its size and therefore remains on one side of the nanopore while the linker part is located in the nanopore. When the applied voltage is reduced and the electric field effect is weakened, the nucleotide or its enzymatically reacted pyrophosphate part will exit the nanopore in the second direction described above. During exit, the intact nucleotide and pyrophosphate will give distinguishable current characteristics to help determine whether the exit group on the linker is the intact nucleotide or the enzymatically reacted product pyrophosphate. By recording the different characteristic currents flowing through the nanopore, the method of the present invention can accurately identify the type of nucleotide passing through the nanopore and determine whether the nucleotide is captured by nucleic acid polymerase and involved in nucleic acid synthesis. The method records the current values in the fully open state of the nanopore; the current characteristic values of nucleotides (both nucleotides and deoxynucleotides) interacting with linker and nanopore at a high voltage, for example, not less than 160 mV, not less than 170 mV, not less than 180 mV, not less than 190 mV, not less than 200 mV; and the current characteristics values of linker carrying the nucleotide/or pyrophosphate exiting the nanopore in the second direction at a low voltage, for example, not higher than 100 mV, not higher than 90 mV, not higher than 80 mV, not higher than 70 mV, not higher than 60 mV, not higher than 50 mV.

The type of nucleotide passing through the nanopore and whether it successfully participates in nucleic acid synthesis is deduced through the logical relationship of the above-mentioned current value with time and system state. The method has great potential and value in reducing deletion errors and insertion errors in nucleic acid sequencing using a nanopore, thereby improving sequencing accuracy.

The electrolyte solution used in the present invention is an electrolyte solution suitable for performing nucleic acid synthesis, such as a KCl solution, specifically a KCl solution with a concentration of 0.1 to 1 M, such as 0.3M KCl solution, 0.5M KCl solution. Under the pH conditions of the electrolyte solution of the present invention, the nucleotide part of the complex of the present invention is negatively charged and can reach the trans side (the second compartment) from the cis side of the phospholipid membrane (the first compartment) through the nanopore along the potential gradient in the electric field. When the nucleotide part reaches the trans side, it can be captured and utilized by the nucleic acid polymerase coupled to the nanopore through free diffusion. And the nucleotide part may interact with the nanopore as it enters/passes through the nanopore thereby generating a characteristic blocking current and is recorded by a microcurrent measurement system connected to the nanopore, thereby determining the base type of this nucleotide being transported by the nanopore.

The present invention provides a way to allow the phosphonucleotide part (or pyrophosphate part, if the synthesis reaction has been carried out) that has passed through the nanopore to the trans side of the phospholipid membrane to return to the cis side through the nanopore again and thus be detected by the nanopore a second time. In some embodiments of the present invention, the nucleotide phosphates are linked by PEG or similar long chains, and a biotin/biotin-binding protein complex. Due to the size much larger than the nanopore aperture, the biotin/biotin-binding protein complex cannot pass through the nanopore but remains on the cis side and confines the nucleotide part near the opening on the trans side of the nanopore by the PEG-linking chain spanning the nanopore. When the electric field force is weakened, the nucleotide near the opening on the trans side of this nanopore will pass through the nanopore again under thermal movement or diffusion and return to the cis side and generate the characteristic blocking current for the second time. Depending on whether the second characteristic blocking current corresponds to an intact nucleotide or to pyrophosphate after the synthesis reaction is complete, it can be used to determine whether this nucleotide is involved in the nucleic acid synthesis reaction.

The present invention relates to a way of covalently coupling a nucleic acid polymerase to a nanopore on the trans side, thereby helping to limit the distance between the polymerase active center and the opening on the trans side of the nanopore to the range of 1 to 3 nm. After the phosphate nucleotide enters the trans side through the nanopore, the phosphate nucleotide is also confined nearby by the long chain of linker, which corresponds to a great increase in the effective local concentration of polymerase and nucleotide, thus greatly reducing the time for the phosphate nucleotide to be captured by the polymerase and contributing to the sequencing efficiency of the present invention.

In addition to using the characteristic blocking current to determine the base type of the nucleotide being transported by the nanopore, the present invention also allows inferring whether the transported nucleotide is involved in DNA synthesis. As mentioned earlier, phosphonucleotides that are transported to the trans side by the nanopore are confined near the nanopore opening by the long chain of linker. After a certain time, the nucleotide (or pyrophosphate if the nucleotide participates and completes the nucleic acid synthesis reaction) eventually passes back to the cis side through the nanopore again by diffusion, regardless of whether it participates in the synthesis reaction or not. If this nucleotide is not involved in the synthesis reaction, then the system will again record the characteristic blocking current corresponding to the exit of the intact nucleotide from the nanopore; conversely, the system will detect the blocking current caused by the exit of pyrophosphate from the nanopore.

In summary, the present invention is implemented so that each nucleotide involved in a nucleic acid synthesis reaction must be transported through the nanopore and its characteristic blocking current is recorded, so as to obtain the information of its base type. After a certain period of time, the molecules transported or entering the trans side pass through the nanopore again and are transported back to the cis side, and in this process, it is determined whether the nucleotide participates in the synthesis reaction. By monitoring each nucleotide that may come into contact with the polymerase and inferring whether it is involved in the reaction, the present invention can obtain the sequence of the template DNA (or RNA) to be analyzed and effectively avoid deletion errors and insertion errors during the sequencing process, obtaining an increase in accuracy.

The present invention is further illustrated by the following examples, which should not be construed as limiting.

### Examples

### Example 1 Design and preparation of the complex of nucleotide-linker-bulky steric hindrance part

In this example, the linker part was chosen to use a long chain polypeptide. One end of the polypeptide was attached to the third phosphate group of the deoxynucleotide triphosphate dNTP and the other end was attached to the maleimide. Different nucleotides of A, T, C and G were attached to different sequences of the polypeptide to facilitate the determination of the base type based on different characteristic blocking current values.

The bulk steric hindrance protein part was the globular protein xylanase, which is structurally stable. The nucleotide-linker-bulky steric hindrance part complex was synthetically prepared by introducing a cysteine residue at the C-terminus of xylanase through protein recombination, and its sulfhydryl-SH formed a stable covalent bond with the maleimide of the linker part.

### Example 2 Preparation of phospholipid bilayer with nanopore

The compartment system that meets the requirements of the present invention was purchased from Warner Instruments, and the aperture size between the left and right compartments was chosen to be 150 µm. The hydrophobic material near the opening was suitable for phospholipid adhesion. A 0.3 M solution of KCl was added to each of the left and right small compartments with the liquid level below the opening. 1,2-diphytanoyl-sn-glycero-3-phosphocholine (DphPC) was selected and dissolved in the organic solvent pentane at a final concentration of 10 mg/ml. This phospholipid solution was added to the existing KCl solution in the compartment to form an organic phase that covered the KCl liquid surface. By adding 0.3 M KCl solution along the outer wall of the compartment, the organic phase containing the phospholipid layer rose with the liquid level thereby flooding the small pore between the two compartments. During this process phospholipids bound to hydrophobic materials near the pore and spontaneously formed a phospholipid bilayer across the pore.

The nanopore solution was added to the first compartment to a final concentration of 1 nM, while testing the current between the two compartments. When a single-molecule nanopore was spontaneously inserted into the phospholipid bilayer, the system would detect the nanopore opening current. The excess nanopore solution was removed in time to ensure that only a single-molecule nanopore channel was present in the phospholipid membrane.

Cysteine was introduced at the trans side of the above nanopore. After the nanopore was successfully embedded in the phospholipid membrane, it was coupled together with DNA polymerase which also carried cysteine, using maleimide-PEG-maleimide (available from the reagent company nanosoft polymers) as a linkage intermediary. The coupled polymerase as well as the corresponding DNA template to be analyzed and primer components were in the second compartment.

The phospholipid membrane with nanopore and DNA replication components (without nucleotides) were prepared by the above operation.

### Example 3 Determination of standard current characteristic value and acquisition of standard current characteristic profile

The nucleic acid sequencing device as described in FIG. 8 was fabricated according to the steps described in Example 2, 0.3 M KCl buffer was added to the first and second compartments, and the current characteristic value Iₒ through the phospholipid bilayer membrane was measured when the nanopore was fully open.

A 5 µM concentration of dATP-L_{A}-B complex prepared as described in Example 1 was added to the first compartment at a voltage value of 180 mV, while the change in current value through the nanopore was recorded at a sampling frequency of 1 KHz or more. In this process, the system first recorded the open nanopore Iₒ, and then the dATP part passed through the nanopore under the electric field from the first compartment into the second compartment, and remained relatively stable under the electric field driving force. At this point the system recorded the current I_{A1} formed by the dATP-L_{A}-B complex in interaction with the nanopore; after 400 ms the voltage value was reduced to 80 mV and continued for 200 ms to record the characteristic current profile I_{A2} caused by the exit of dATP from the nanopore in the direction from the second compartment to the first compartment. The above voltage stimulation protocol was repeatedly used to continuously record current changes for 15 minutes to obtain a sufficiently large sample size to obtain a range of characteristic blocking current values I_{A1} for dATP-L_{A}-B at 180 mV, as well as to clarify the reproducibility of the characteristics presented by the current profile I_{A2}.

The ranges of the respective values of the standard current characteristic values I_{T1}, I_{C1}, and I_{G1} corresponding to dTTP, dCTP, and dGTP were determined in the same way, and the characteristic current profiles I_{T2}, I_{C2}, and I_{G2} when the corresponding nucleotides exited the nanopore were recorded, respectively.

The characteristic current profiles I_{A3}, I_{B3}, I_{C3} and I_{G3} of the four complexes, PP-L_{A}-B, PP-L_{T}-B, PP-L_{C}-B and PP-L_{G}-B, during the exit of the PP part from the second compartment back to the first compartment at 80 mV were recorded in the above manner.

### Example 4 Determination of nucleic acid sequence

The nucleic acid sequence to be analyzed was synthesized by Tsingke Biotechnology Co., Ltd., Beijing, as described in SEQ ID NO. 1,
SEQ ID NO. 1:
   5' ATAGACGCGGCCAAATTACGGCCGAT 3'
   where the underlined part is the complementary sequence of the binding primer. Primer sequence is as described in SEQ ID NO. 2.
SEQ ID NO. 2:
   5' ATCGGCCGTAATTTGGCC 3'

In the sequencing device as shown in FIG. 8, the nucleic acid sequence to be analyzed and the primer sequence were dissolved in a 0.3 M KCl solution and added to the second compartment of the nucleic acid sequencing device, where the nucleic acid polymerase complex was coupled to the nanopore thereby being near the opening on the trans side of the nanopore in the second compartment.

The nucleotide complexes prepared as described in Example 1 were dissolved in a 0.3 M KCl solution and added to the first compartment of the nucleic acid sequencing device.

First cycle:
applying a positive electric field V₁ = 180 mV directed from the first compartment to the second compartment for a duration t₁ = 400 ms and recording the current change,
applying a positive electric field V₂ = 80 mV directed from the first compartment to the second compartment for a duration t₂ = 200 ms, and recording the current change,
applying a reverse electric field V₃ = -180 mV directed from the second compartment to the first compartment for a duration t₃ = 5 ms to ensure that all macromolecules exitted the nanopore completely;

### Second cycle:

### Until end of sequencing.

Based on the current variation graphs obtained for each cycle described above, the measured current values were compared to the individual standard current characteristic values measured in Example 3 to determine the type of nucleotide that crossed the nanopore in each cycle, as well as to determine whether the nucleotide was successfully synthesized onto the nucleic acid sequence to be analyzed.

Based on the above analysis and judgment, it was determined that the nucleotides involved in the synthesis reaction were GCGTCTAT in order, so the original nucleic acid sequence to be analyzed was ATAGACGC from 5' to 3'.

The present invention is not limited to the particular embodiments described in this application, which are intended as a single illustration of various aspects of the present invention. Many modifications and variations of the invention can be made without departing from the spirit and scope of the invention, as will be apparent to those skilled in the art. Functionally equivalent methods and apparatuses within the scope of the present invention, other than those enumerated herein, will become apparent to those skilled in the art from the foregoing description. These modifications and variations are intended to fall within the scope of the appended claims. The invention is limited only by the terms of the appended claims and the full scope of the equivalents of those claims. It should be understood that the present invention is not limited to particular methods, reagents, compound compositions, or biological systems that are of course subj ect to variation. It should also be understood that the terms used herein are intended to describe particular embodiments only and are not limiting.

## Claims

1. A method for determining type of a nucleotide on a nucleic acid sequence to be analyzed, comprising the steps of:
S01 providing at least one nucleotide molecule in a first compartment and a nucleic acid sequence to be analyzed in a second compartment, with the first compartment and the second compartment separated by a membrane having at least one nanopore;
S02 applying an electric field to drive the nucleotide molecule and/or a part thereof to pass through the nanopore in a first direction or to be inserted in the nanopore;
S03 measuring a first current characteristic value passing through the membrane in the state of S02 to identify the nucleotide molecule and/or a part thereof;
S04 applying an electric field in a direction opposite to that of the electric field applied in step S02 or in the same direction but with a lower driving voltage, causing the nucleotide molecule and/or a part thereof to pass through the nanopore and/or exit the nanopore in a second direction, with the second direction being opposite to the first direction;
S05 measuring a second current characteristic value passing through the membrane to identify the nucleotide molecule and/or a part thereof, comparing the second current characteristic value with a pre-determined standard current characteristic value of the nucleotide molecule or polyphosphate molecule in the state of S04 to determine whether the nucleotide molecule and/or a part thereof is attached to the nucleic acid sequence to be analyzed, and thus determining the type of nucleotide on the nucleic acid sequence to be analyzed in the second compartment.

2. The method according to claim 1, wherein the nucleotide molecule is a modified nucleotide molecule as shown in Formula I.
NXP-L-B Formula I
wherein NXP represents a phosphate deoxyribonucleotide and/or a phosphate nucleotide, the phosphate deoxyribonucleotides comprising dAXP, dTXP, dCXP and dGXP; the phosphate nucleotides comprising AXP, TXP/UXP, CXP and GXP.
L represents a linker part having a long chain segment and a linking segment.
B represents a bulky steric hindrance part, which is a protein molecule, and is connected to the linker part through the linking segment.

3. The method according to claim 1, wherein the membrane is selected from a natural lipid bilayer or a bilayer membrane formed by artificial amphiphilic molecules.

4. The method according to claim 1, wherein the nanopore is selected from a natural protein nanopore or a nanopore prepared from artificial material.

5. The method according to claim 1, wherein a nucleic acid polymerase and a template primer are further provided in the second compartment.

6. A sequencing method for a nucleic acid sequence to be analyzed, comprising repeating steps S01 to S05 of the method according to claim 1, sequentially determining type of each nucleotide of the nucleic acid sequence to be analyzed until the sequencing of the nucleic acid sequence to be analyzed is completed.

7. A nucleic acid sequencing device for the method according to claim 1 or 6, comprising:
(a) a first compartment and a second compartment separated by a membrane having at least one nanopore;
(b) means for applying an electric field across the membrane; and
(c) components used to measure a current flowing through the membrane.
